# EUROPEAN PATENT SPECIFICATION

(11) **EP 2 499 914 B1**
(45) Date of publication and mention of the grant of the patent: **20.12.2017**
(21) Application number: 12158959.2
(22) Date of filing: 10.03.2012
(51) Int. Cl.: A01N 47/44, A01N 25/00, A01N 25/02, A61K 31/155, A01P 1/00

(54) **Antiseptic solutions comprising di-(4-chlorophenyldiguanidino)-hexane and an anionic dye and their preparation**
Antiseptische Lösung etnhaltend Di-(4-Chlor-Phenyldiguanido)-Hexan und ein anionischer Farbstoff und ihre Herstellung
Solution antiseptique d'un composé de di(4-chlorophényldiguanido)-héxané et son procédé de préparation

(30) Priority: 20.06.2011 ES 201131038; 24.06.2011 US 201161500969 P; 17.08.2011 US 201113211846; 14.03.2011 EP 11382069
(43) Date of publication of application: 19.09.2012
(73) Proprietor: Medichem, S.A., 08970 Sant Joan Despi, Barcelona (ES)
(72) Inventor: Lloret Perez, Sergio, Barcelona 08030 (ES); Puigvert Colomer, Marina, 08014 Barcelona (ES)
(74) Representative: CMS Cameron McKenna Nabarro Olswang LLP

(56) References cited:
- EP-A1- 1 044 686
- US-A- 4 548 807
- US-A1- 2003 059 379
- US-A1- 2007 254 854
- DATABASE WPI Week 200910 Thomson Scientific, London, GB; AN 2009-E25324 XP002657092, -& CN 101 336 910 A (GUANGZHOU OUTIKE DISINFECTANT CO LTD) 7 January 2009 (2009-01-07)
- "ChloraPrep with Tint Three-Year Exclusivity and Related Issues", FDA - Dockets, 3 August 2006 (2006-08-03), pages 1-27, XP055069031, USA Retrieved from the Internet: URL:http://www.fda.gov/ohrms/dockets/docke ts/05p0458/05p-0458-ts00001.pdf [retrieved on 2013-07-01]

## Description

The present invention is concerned with an antiseptic solution comprising a di(4-chloro-phenyldiguanido) derivative, and an improved process of preparing the same.

### BACKGROUND OF THE INVENTION

Chlorhexidine is the international common accepted name for *N,N-*bis(4-chlorophenyl)-3,12-diimino-2,4,11,13-tetraazatetradecanediimidamide (also known as 1,6-di(4'-chlorophenyldiguanido)hexane), and has an empirical formula of C₂₂H₃₀Cl₂N₁₀, a molecular weight of 505.45 g/mol, and a chemical structure of formula (I):

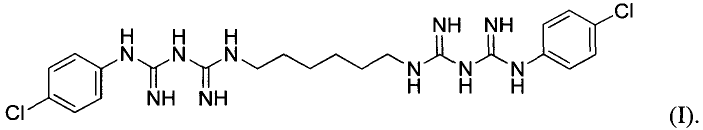

Chlorhexidine is a well-known topical antiseptic and disinfectant that has been used for more than 30 years. Products comprising chlorhexidine are used for a number of applications such as, for example, daily hygiene application, oral antiseptic applications, hand and skin disinfection, and general disinfection (e.g., equipment, surfaces and textiles). Chlorhexidine is a highly active bactericidal agent having a broad activity with low levels of toxicity and strong skin binding properties. Chlorhexidine is primarily bactericidal against gram-positive and gram-negative bacteria, and its activity extends to yeasts, including *Candida albicans.* The bactericidal activity of chlorhexidine differs from that observed with povidone-iodine or 70% isopropyl alcohol. For example, chlorhexidine has demonstrated an immediate bactericidal effect as well as a cumulative effect that persists for hours and even days after it is applied, and unlike iodophors, the germicidal activity of chlorhexidine has shown statistically significant efficacy in reducing bacterial counts in the presence of blood and other protein-rich biomaterials.

Chlorhexidine is often applied preoperatively to disinfect materials and surfaces or skin of a patient prior to surgery. An important consideration for the user is the appearance of a chlorhexidine solution. For example, upon visual inspection, the user needs to be able to determine that a) the antiseptic solution has been applied, and b) where the antiseptic solution has been applied. However, chlorhexidine by itself forms clear, colorless solutions which makes it difficult for the user to make such a determination. Thus, it is desirable to use colored chlorhexidine solutions.

Numerous problems are encountered when color additives (e.g., tints or dyes) are added to an antiseptic solution of chlorhexidine in amount sufficient to stain a patient's skin or surgical field before surgery. The tint or dye can react with chlorhexidine to form a precipitate, which can lead to non-uniform distribution of the colored antiseptic solution upon its application. In addition, the coloring additive can react with chlorhexidine to form impurities thereby partially inactivating the chlorhexidine solution and increasing the impurity profile of the antiseptic solution. In addition, the chemical stability of the product is lessened resulting in a shortened shelf life.

The development of colored solutions of chlorhexidine, with a sufficient shelf life has proven to be difficult. Moreover, the development of colored solutions of chlorhexidine, advantageously prepared by efficient formulation processes has proved problematic.

U.S. 2004/0179889 ("the '889 publication") acknowledges the problems associated with colored chlorhexidine solutions. The '889 publication purportedly describes that the problems can be alleviated by using a specific, specialized applicator for applying chlorhexidine solution. The disclosed applicator comprises at least one ampoule formed of a frangible material and adapted to contain liquid to be applied; at least one hollow body defining an internal chamber adapted to receive at least one ampoule; and at least one porous element that contains colorant, wherein the porous element is positioned such that liquid flows through the porous element when at least one ampoule is fractured and colorant is transferred to the liquid to be applied.

The use of specific applicators to administer antiseptic solution or to reconstitute the solution prior to its use, is inconvenient and is not feasible for some applications.

WO 2007/130981 ("the '981 publication") supposedly discloses a solution of chlorhexidine together with cationic dye in an amount sufficient to stain patient's skin. It is believed that since chlorhexidine pharmaceutically acceptable salts are cationic compounds, they are compatible with other cationic and non ionic substances, but are chemically incompatible with anionic compounds.

The use of cationic dyes, as well as products comprising cationic dyes, can pose a health hazard. Numerous disadvantages are related to using cationic dyes in antiseptic formulations, including adverse toxicological and carcinogenic effects. Reports of hypersensitivity and hyperkinetic activity, especially among children, add to concerns over the safety of using cationic dyes in products.

Moreover, the '981 publication describes that an anionic dye may be used provided that the antiseptic solution also comprises a cationic excipient. The '981 publication also describes that anionic dyes, including FD&C dyes, form a precipitate with chlorhexidine, even at very low concentrations. The precipitate forms as an insoluble salt of a chlorhexidine cation and at least one dye anion. As such, adding an anionic dye alone to an aqueous chlorhexidine solution removes a significant fraction of the chlorhexidine from solution, thereby decreasing the efficacy of the solution.

WO 2007/130982 ("the '982 publication") attempts to solve the above described problems by providing solutions of chlorhexidine together with an anionic dye and a cationic excipient (e.g., cationic detergents, surfactants or excipients containing quaternary nitrogen. The '982 publication describes the use of a cationic excipient together with an anionic excipient is essential and that as the negative charge of an anionic dye is "hidden" from the chlorhexidine by a cationic excipient, the chlorhexidine-dye salt will not immediately form.

However, using additional excipients in antiseptic solution, especially cationic excipients may cause dangerous adverse affects related with allergies, intolerance and/or irritation.

In addition, according to the Summary of Product Characteristics for CHLORAPREP® 2% (w/v)/70% (v/v) cutaneous solution, chlorhexidine is incompatible with anionic agents (e.g., http://www.medicines.org.uk/emc/medicine/22302/SPC/ chloraprep/).

CN 101336910 discloses a composition comprising a slat of chlorhexidine (0.5-5%), at least one colorant (0.5-1.5%), ethanol (0.5-1.5%) or isopropanol and water (balance). The composition is prepared by dissolving 20% chlorhexidine gluconate with 10 kg of pure water, mixing and stirring for 5 minutes; adding 70 L of 99.9% ethanol, mixing and stirring for 5 minutes, adding 0.8 kg of sunset yellow and moderate humectant, stabilizing agent and pure water, stirring for 10 minutes; examining, filing and encapsulating. EP1044686 discloses a solution for cutaneous application that contains chlorhexidine gluconate and ethanol (as active agents), plus a mixture of quinoline yellow and patent blue (as colorants) to give the solution a green color when applied to the skin. The method of manufacturing of said solution comprises the following successive phases, the unit amount of solution corresponding to 500 g:one starts with the preparation of dyes, that is to say is mixed with 142 ml demineralized water, 250 mg of yellow quinoline and 50 mg of patent blue, then the mixture was stirred for 10 minutes; to the mixture is added 422 ml of 95% ethyl alcohol and 15 g of chlorhexidine; whole stirred for 10 minutes; one proceeds to a clarifying filtration. US 2003/0059379 discloses methods for treating periodontal disease and other diseased tissue that utilizes a dye composition and laser energy. The dye composition may include 78.88% water, 0.5%D&C red #40, 20% ethanol, 0.12 chlorhexidine gluconate and 0.5% peppermint oil. US 4,548,807 discloses a method, composition and containerized product for use in preventing the disease of mastitis in dairy cows and other animals. The invention employs a homogenous disinfectant composition in the form of an aerosol spray, and it may contain 0.4% chlorahexidine acetate, 35.0% dimethyl ether, 10.0% ethyl alcohol, 9.83 % glycerine, 0.003% FD&C Blue No. 1 and 44.767% water. A presentation "ChloraPrep® with Tint Three Year Exclusivity and Related Issues" FDA Dockets (2006-08-03) discloses the product ChloraPrep® with Tint which comprises chlorhexidine gluconate 2% and isopropyl alcohol 70%. The product includes an over-the-counter topical sponge and is used as an antiseptic for surgical prepatration. FD&C Green No. 3 is disclosed as a tint ingredient.

In view of the foregoing, there is a need to provide colored antiseptic solutions comprising chlorhexidine which are safe, have improved properties and good chemical stability. There is also a need for colored antiseptic chlorhexidine solutions which can be prepared by efficient formulation processes.

### BRIEF SUMMARY OF THE INVENTION

The present invention provides colored, stable antiseptic solution comprising a di(4-chlorophenyldiguanido) compound of formula (I) or a pharmaceutically acceptable salt thereof, one or more anionic dyes, and one or more solvents, wherein the compound of formula (I) or a pharmaceutically acceptable salt thereof is present in an amount of 1% and 3% (w/v) and the one or more anionic dyes is present in an amount of 0.01% to 0.04% (w/v), the one or more solvents comprises water and from 20 to 95% (v/v) of one or more C₁₋₈ alkanols wherein the antiseptic solution contains less than 0.01 wt% of cationic excipients, wherein the stable antiseptic solution is stable for at least 6 months at 25 ± 2 °C and 75% ± 5% relative humidity, and wherein the stable antiseptic solution is prepared by the process according to claim 3.

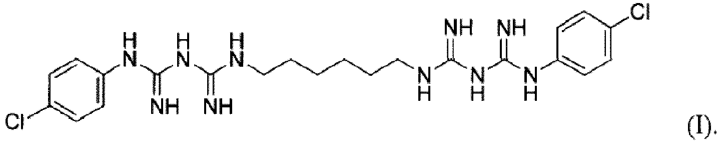

The antiseptic solution of the invention is suitable for disinfecting the skin or surgical field prior to invasive medical procedures, and the antiseptic solution of the invention has good pharmacokinetic properties, including a rapid onset of action and long lasting activity against pathogens.

The stable antiseptic solution of the invention exhibits a high stability and excellent compatibility with anionic dyes, even being substantially free of cationic excipients. Accordingly, the antiseptic solutions of the invention is not contra-indicated in patients having a hypersensitivity to cationic excipients or cationic dyes. Moreover, the substantial absence of cationic excipients reduces the absorption of isopropyl alcohol or chlorhexidine through skin. Since there is a little percutaneous absorption of the antiseptic solution of the invention it is indicated, for example, for use on pre-injection sites.

The invention also provides a process for preparing colored, stable antiseptic solution comprising a di(4-chlorophenyldiguanido) compound of formula (I) or a pharmaceutically acceptable salt thereof (as defined above) said process comprising: (i) dissolving one or more anionic dyes in one or more first solvents to provide an anionic dye solution, wherein the first solvent comprises water and optionally further comprises one or more C₁₋₈ alkanols, (ii) dissolving the di(4-chlorophenyldiguanido) compound of formula (I) or a pharmaceutically acceptable salt thereof in one or more second solvents to provide a solution of a compound of formula(I) or a pharmaceutically acceptable salt thereof, wherein the second solvent is selected from the group consisting of one or more C₁₋₈ alkanols, water, and mixtures thereof, and (iii) combining the anionic dye solution and solution of a compound of formula (I) or a pharmaceutically acceptable salt thereof, with the proviso that if the solvent for the anionic dye solution is only water, then the one or more second solvents comprises one or more C₁₋₈ alkanols.

### DETAILED DESCRIPTION OF THE INVENTION

The present invention provides colored, stable antiseptic solutions as defined in the claims, comprising a di(4-chlorophenyldiguanido) compound of formula (I), or a pharmaceutically acceptable salt thereof, wherein the antiseptic solution contains less than 0.01 % of cationic excipients.

Without wishing to be bound to a particular theory, it is believed that the antiseptic solution of the invention exerts its biological effects upon bacterial cells through nonspecific interactions with acidic phospholipids of the cell membranes. The antiseptic solution of the invention is both an excellent antiseptic and disinfectant. Although the antiseptic solution of the invention is more effective against gram-positive bacteria, the antiseptic solutions of the invention exhibit bactericidal or bacteriostatic activity against a wide range of gram-positive and gram-negative bacteria. The antiseptic solutions of the invention also exhibit some activity against certain species of Pseudomonas and Proteus, as well as some viruses and fungi. It is believed that the combination of solvents (e.g., isopropyl alcohol and water) used to prepare the antiseptic solutions of the invention can enhance both efficacy and stability of the antiseptic solutions.

In accordance with the invention, the antiseptic solution is preferably free of cationic excipients. Applicants have found that antiseptic prepared according to the process of claim 3 comprising chlorhexidine or a pharmaceutically acceptable salt thereof, one or more anionic dyes, and one or more solvents exhibit good chemical and physical stability even though said solutions comprises less than 0.01 wt %, preferably less than 0.001 wt % of the cationic excipient. In a preferred embodiment the antiseptic solution does not comprise any cationic excipient or only in amounts under the detection limit. In a particularly preferred embodiment, the antiseptic solution of the invention is free of any cationic excipients.

In accordance with the invention, the antiseptic solution of the invention comprises one or more anionic dyes. Although others have reported adding colorants to di(4-chlorophenyldiguanido) compound of formula (I), numerous problems are encountered with an increased amount of colorants. However, it is believed that applicants are the first to report the addition of anionic dye in amount sufficient to stain or color, without the need of further additional excipients to provide an antiseptic solution of chlorhexidine which does not precipitate and is chemically stable for at least 6 months..

Applicants have surprisingly discovered that antiseptic solutions of a di(4-chlorophenyldiguanido) compound of formula (I) or a pharmaceutically acceptable salt thereof can be prepared wherein the solution comprises one or more anionic dyes even though the antiseptic solution comprises less than 0.01% of cationic excipients. Without wishing to be bound to a particular theory, it is proposed that the inclusion of one or more anionic dyes into the antiseptic solution of the present invention can prevent precipitation and can assure chemical stability and shelf life of the antiseptic solutions. The antiseptic solutions of the invention are stable for at least 6 months, at least 12 months, and even at least 18 months. Moreover, the antiseptic solution of the present invention is stable in relative extreme hot or cold temperature conditions.

As used herein, the term "antiseptic solution" refers to any liquid which comprises said di(4-chlorophenyldiguanido) compound of formula (I) (i.e., chlorhexidine) or a pharmaceutically acceptable salt thereof wherein all the components are completely dissolved and said solution is ready for administration. Further, the antiseptic solution does not require either any further preparation (e.g., reconstitution) prior to administration, or any specialized applicator. The antiseptic solution of the invention is suitable as an antibacterial and/or disinfectant solution for skin disinfection; handwashing; oral care; irrigation of surgical wounds, the urinary bladder or vagina; topical treatment of burn wounds; for treatment of peritonitis in peritoneal dialysis; and/or for all the purposes related to its antiseptic and/or disinfectant properties to prevent infections such a nosocomial infection, surgical-site infections, catheter-related infections, surgical wound infections, oral infections.

As used herein, the term "stable" refers to a solution that after 6 months is clear and leaves no residual solids visible to the human eye. Moreover, the stable antiseptic solution of the invention is clear and does not leave residual solids visible to the human eye after a stability test in the conditions of 25 ± 2 °C and 60% ± 5% relative humidity during at least 6 months, even more preferably during at least 12 months and the most preferably during at least 18 months and is chemically stable, i.e., contains insubstantial amount of *p*-chloroaniline impurity, preferably less than 0.25% (w/w), after undergoing the stability test mentioned above.

As used herein, the term "anionic dye" refers to any colored substance, for example, those containing auxochromes, and thus capable of coloring substances to which they are applied or incorporated. Such colorants are used, for example, for staining and coloring, as a test reagent, and as a therapeutic agent. Anionic dyes are characterized that they have a negative charge and attach to cationic surfaces. Anionic dyes include many compounds from various classes of dyes having differences in structure (e.g., azoic, anthraquinone, triphenylmethane and nitro dyes) but possess ionic substituents as a common, water-solubilizing feature.

The examples of "cationic excipients" can be found in the WO 2007/130982. Cationic dyes are also considered as cationic excipients in the context of the present invention.

As used herein, the term "essentially free" refers to an insubstantial amount, for example, when describing an amount of cationic excipient, essentially free refers to less than 0.01% by weight of cationic excipient, or even less than 0.001 % by weight of cationic excipient. Preferably the antiseptic solution is free of any cationic excipients.

As used herein, the term "insubstantial" amount of *p*-chloroaniline impurity means preferably less than approximately 0.25% (w/w) (i.e., not more than 0.25%; NMT 0.25%) after the above mentioned stability test. The stability studies are preferably carried out as defined in ICH Topic Q1A "Stability Testing of New Drug Substances and Products" (CPMP/ICH/2736/99).

As used herein, the term "surgical field" refers to a sterile, isolated area of the operative field, where surgery is performed, including but not limited to the patient and surfaces surrounding the patient (e.g., operating table and surgical equipment).

As used herein, the terms "lower alcohol" or "lower alkanol" refer to straight chain or branched alkyl residues containing 1 to 8 carbon atoms with at least one hydroxy group, such as methanol, ethanol, propanol, *n*-propanol, isopropanol, butanol, *n*-butanol, 1-butanol, 2-butanol, *tert*-butanol, pentanol and the like.

As used herein, precipitation and/or particulate contamination refer to the formation of any solids in the antiseptic solution of the invention or the particulate contamination that consists of extraneous, mobile, undissolved particles, other than gas bubbles, unintentionally present in the solution. Precipitation or particulate contamination can be measured, for example, by any suitable tests that provide an assessment of the quality of the solution comprising di(4-chlorophenyldiguanido) compound of formula (I) or pharmaceutically acceptable salt thereof and an anionic dye in an amount sufficient to stain a patient's skin or surgical field before surgery. An illustrative test is a visual assessment for visible particles. For example, precipitation and/or particulate contamination of the antiseptic solution of the invention can be measured by first removing any adherent labels from the container and washing and drying the outside of the container, if the container is transparent. If the container is not transparent the antiseptic solution can be placed in previously washed and dried transparent container. Further, the container might be gently swirled or inverted, ensuring that air bubbles are not introduced, and then the contents of the container should be observed. It is desirable that no particulate contamination is observed. One skilled in the art will recognize that other test methods are suitable.

As used herein, the chemical stability during shelf life refers to the antiseptic solution of the invention having a desirable impurity profile as described herein. Typical impurities include, for example, *p*-chloroaniline and visible particles. Desirably, the antiseptic solution of the invention has a low level of impurities and is stable over a period of time (e.g., 6 months, 12 months, and/or 18 months). For example, desirably the antiseptic solution of the invention has and maintains a level of *p*-chloroaniline that is not more than 0.25% upon storage for 6 months at either room temperature (e.g., 20-25 °C) or at 40 °C. Further, it is desirable that the colored, antiseptic solution of the invention remains free of visible particles upon storage and during use. Any suitable method known to one of ordinary skill can be used to test for impurities. In a preferred embodiment, a method to measure *p*-chloroaniline impurity is an HPLC method, based on USP33 references and ICH guidelines requirements.

In keeping with the invention, the antiseptic solution of the invention comprises one or more anionic dyes. Suitable anionic dyes that may be employed within antiseptic solution of the present invention include, but are not limited to, FD&C dyes, such as, for example, FD&C Blue No. 1 (Brilliant Blue FCF), FD&C Blue No. 2 (Indigo Carmine), FD&C Green No.3 (Fast Green FCF), FD&C Red No.3 (Erythrosine), FD&C Red No. 40 (Allura Red), Food Red 3 (Carmoisine), FD&C Yellow No.5 (Tartrazine), and FD&C Yellow No. 6 (Sunset Yellow FCF). Preferably, the antiseptic solution of the present invention comprises FD&C Red No. 40 (Allura Red) anionic dye. In an embodiment, the one or more anionic dyes of the antiseptic solution is selected from the group consisting of FD&C Blue No.1, FD&C Blue No.2, FD&C Green No.3, FD&C Red 40, Food Red 3 (Carmoisine), FD&C Yellow No.5, and FD&C Yellow No.6, and mixtures thereof. These anionic dyes are also known as: FD&C Blue No. 1, CAS 3844-45-9, Blue FCF, Acid Blue 9, Alzen Food Blue No. 1, Atracid Blue FG, Erioglaucine, Eriosky blue, Patent Blue AR, Xylene Blue VSG, FD&C Blue No. 2, CAS 860-22-0, Indigo carmine, 3,3'-dioxo-2,2'-bis-indolyden-5,5'-disulfonic acid disodium salt, indigotine, 5,5'-indigodisulfonic acid sodium salt FD&C Green No. 3, CAS 2353-45-9, Fast Green FCF, Food green 3, Green 1724, Solid Green FCF, C.I. 42053, ethyl-[4-[[4-[ethyl-[(3-sulfophenyl) methyl] amino] phenyl]-(4-hydroxy-2-sulfophenyl) methylidene]-1-cyclohexa-2,5-dienylidene]-[(3-sulfophenyl)methyl] azanium, FD&C Red No.3, CAS 16423-68-0, erythrosine, 2-(6-hydroxy-2,4,5,7-tetraiodo-3-oxo-xanthen-9-yl)benzoic acid, FD&C Red No. 40, CAS 25956-17-6, Allura Red, Food Red 17, C.I. 16035, E129, 2-naphthalenesulfonic acid disodium salt, disodium 6-hydroxy-5-((2-methoxy-5-methyl-4-sulfophenyl)azo)-2-naphthalenesulfonate Food Red 3: CAS 3567-69-9, azorubine, carmoisine, disodium 4-hydroxy-2-[(E)-(4-sulfonato-1-naphthyl)diazenyl]naphthalene-1-sulfonate FD&C Yellow No.5, CAS 1934-21-0, tartrazine, trisodium (4E)-5-oxo-1-(4-sulfonatophenyl)-4-[(4-sulfonatophenyl)hydrazono]-3-pyrazolecarboxylate FD&C Yellow No.6, CAS 2783-94-0, Orange Yellow S, FD&C Yellow 6, C.I. 15985, E110, disodium 6-hydroxy-5-[(4-sulfophenyl)azo]-2-naphthalenesulfononate.

In a particularly preferred embodiment, the antiseptic solution comprises one anionic dye.

The antiseptic solutions of the invention comprise a suitable amount of anionic dye. Typically, the antiseptic solutions comprise an amount of anionic dye sufficient to stain a patient's skin or surgical field when applied before surgery. The concentration of the anionic dye is in the range of 0.01% (w/v) to 0.04% (w/v) of the solution and even more preferably in the range of 0.02% (w/v) to 0.03% (w/v) of the solution. Most preferably in such embodiments of the invention, the concentration of anionic dye is within the range 0.0225 to 0.0275% (w/v) of the solution.

In keeping with an aspect of the invention, the antiseptic solution of the invention can comprise a pharmaceutically acceptable salt of the di(4-chlorophenyldiguanido) compound of formula (I). A pharmaceutically acceptable salt of chlorhexidine typically is used to help with solubility. In a preferred embodiment, the antiseptic solution of the invention comprises at least one pharmaceutically acceptable salt of di(4-chlorophenyldiguanido) compound of formula (I). Suitable pharmaceutically acceptable salts of the di(4-chlorophenyldiguanido) compound of formula (I) include the gluconate, acetate, chloride, bromide, nitrate, sulfate, carbonate, and phosphanilate salts, and mixtures thereof. In a particularly preferred embodiment, the pharmaceutically acceptable salt is the gluconate salt of di(4-chlorophenyldiguanido) compound of formula (I), that is chlorhexidine gluconate.

One skilled in the art will recognize that chlorhexidine can form bivalent salts (e.g. chlorhexidine diglucnnate).

The concentration of di(4-chlorophenyldiguanido) compound of formula (I) or its pharmaceutically acceptable salt in the antiseptic solution of the invention can vary within various embodiments of the present invention. The concentration of di(4-chlorophenyldiguanido) compound of formula (I) or its pharmaceutically acceptable salt is in the range of 1% to 3% (w/v) and still more preferably in the range of 1.5% to 2.5% (w/v). Even more preferably, the antiseptic solution of the present invention comprises within the range 1.8 to 2.2% (w/v) of the di(4-chlorophenyldiguanido) compound of formula (I) or pharmaceutically acceptable salt thereof.

It is desirable that the one or more solvents of the antiseptic solution is compatible with the other components of the composition and is non-toxic when applied to human or animal skin. Suitable solvents include, but are not limited to water, alcohols, acetone, esters, chlorinated hydrocarbons and chlorofluorohydrocarbons. The solvent of the antiseptic solution comprises a mixture of one or more C₁₋₈ alkanols and water, preferably deionized water. The solvent comprises water and 20 to 95% (v/v) of at least one C₁-C₈ lower alkanol such as, for example, isopropanol, ethanol and other alcohols. Preferably, the solvent comprises water and 50 to 90% (v/v) of at least one C₁-C₈ lower alkanols. The preferred lower alkanol is isopropanol. The preferred water is deionized water. In a preferred embodiment, the solvent of the antiseptic solution of the invention consists essentially of deionized water and within the range 63 to 77% (v/v) of isopropyl alcohol. Preferably the solvent of antiseptic solution of the invention is deionized water and 70% (v/v) of isopropyl alcohol.

Applicants have found that an antiseptic solution of the invention has good stability when the active ingredient di(4-chlorophenyldiguanido) compound of formula (I) or pharmaceutically acceptable salt thereof used to form the antiseptic solution of the invention, has a residual concentration of *p*-chloroaniline of less than 500 ppm, preferably less than 300 ppm, more preferably less than 100 ppm, even more preferably less than 50 ppm, and still even more preferably equal to or less than 43 ppm, by HPLC. Additionally, good results are obtained when di(4-chlorophenyldiguanido) compound of formula (I) or pharmaceutically acceptable salt thereof, as used to form the antiseptic solution of the invention typically shows a total impurities content of less than 0.1%, and preferably less than 0.05%, as determined by HPLC.

In an embodiment, the present invention provides an antiseptic solution as defined in claim 10 consisting of a pharmaceutically acceptable salt of di(4-chlorophenyldiguanido) compound of formula (I), one or more solvents, and one or more anionic dyes in an amount sufficient to stain a patient's skin or surgical field before surgery, wherein the solvent is an aqueous C₁₋₈ alcohol solution. In a preferred embodiment, the antiseptic solution consists of di(4-chlorophenyldiguanido) compound of formula (I) or pharmaceutically acceptable salt thereof, a mixture of isopropanol and water, and one or more anionic dyes in an amount sufficient to stain a patient's skin or surgical field before surgery. In a particularly preferred embodiment, the antiseptic solution consists of 2% (w/v) of di(4-chlorophenyldiguanido) compound of formula (I) digluconate, 70% (v/v) of isopropanol, water and one or more anionic dyes in an amount sufficient to stain a patient's skin or surgical field before surgery.

In a particular preferred embodiment, the invention provides a stable antiseptic solution prepared according to the process of claim 3 consisting of a di(4-chlorophenyldiguanido) compound of formula (I) or a pharmaceutically acceptable salt thereof, and one or more anionic dyes, wherein the anionic dye or the total amount of anionic dyes present in the antiseptic solution are sufficient to stain a patient's skin or surgical field before surgery, wherein the antiseptic solution is free of any cationic excipient, wherein the concentration of the anionic dye is an amount of 0.01% to 0.04%, the di(4-chlorophenyldi-guanido) compound of formula (I) or pharmaceutically acceptable salt thereof is present in an amount of 1 to 3 % (w/v), wherein the solvent of the solution comprises a mixture of one or more C₁₋₈ alkanols and deionized water.

In a first preferred embodiment, the invention provides a stable antiseptic solution comprising within the range 27 to 33% (v/v) of water, within the range 63 to 77% (v/v) of isopropanol, within the range 1.8 to 2.2% (w/v) of a pharmaceutically acceptable salt of the di(4-chlorophenyldiguanido) compound of formula (I), preferably the gluconate salt, and within the range 0.0225 to 0.0275% (w/v) of an anionic dye. In a second preferred embodiment, the invention provides a stable antiseptic solution comprising within the range 27 to 33% (v/v) of water, within the range 63 to 77% (v/v) of isopropanol, within the range 1.8 to 2.2% (w/v) of a pharmaceutically acceptable salt of the di(4-chlorophenyldiguanido) compound of formula (I), preferably the gluconate salt, and within the range 0.18 to 0.22% (w/v) of an anionic dye.

Although the solution of the invention preferably comprises the excipients mentioned herein, and more preferably consists of these excipients, one skilled in the art will recognize that it is possible to add further excipients which do not materially affect the invention. For example, non-ionic excipients can be added such as, for example, alcohols, alkanolamides (amides derived from alkanolamines, cocamide MEA, cocamide DEA, oleamide DEA, fatty acid diethanolamide of vegetable oils, ethoxylated amides, PEG-4-cocamide MEA amine oxides, esters (ethoxylated carboxylic acid-PEG-40-castor oil, ethoxylated glycerides, PEG-24-glyceryl stearate, glycol esters and derivatives, monoglycerides, polyglyceryl esters, esters and ethers of polyols, esters of sorbitan/sorbitol, triesters of phosphoric acid), ethers (ethoxylated alcohols, ceteareth-12, ceteareth-20, ceteareth-30, laureth-2, laureth-3, laureth-4, oleth-5, oleth-10, ethoxylated lanolin derivatives, polysiloxanes ethoxylated, propoxylated ethers of PEG-PPG-1-PEG-9-lauryl ether glycol).

The following abbreviations are used herein: monoethanolamine (MEA); diethanolamine DEA); polyethyleneglycol (PEG); and polypropyleneglycol (PPG).

The present invention further provides a processes of preparing the antiseptic solution described herein. Applicants have surprisingly found that when the antiseptic solution of the present invention is prepared by combining or adding the components in a particular manner (e.g., specific order), precipitation is avoided when compared to other processes. Surprisingly, the stability of the final antiseptic solution depends on the order in which the excipients are combined/mixed.

The process of the invention comprises the following steps: (i) dissolving one or more anionic dyes in one or more first solvents to provide an anionic dye solution, wherein the first solvent comprises water and optionally further comprises one or more C₁₋₈ alkanols, (ii) dissolving the di(4-chlorophenyldiguanido) compound of formula (I) or a pharmaceutically acceptable salt thereof in one or more second solvents to provide a solution of a compound of formula(I) or a pharmaceutically acceptable salt thereof, wherein the second solvent is selected from the group consisting of one or more C₁₋₈ alkanols, water, and mixtures thereof, and (iii) combining the anionic dye solution and solution of a compound of formula (I) or a pharmaceutically acceptable salt thereof, with the proviso that if the solvent for the anionic dye solution is only water, then the one or more second solvents comprises one or more C₁₋₈ alkanols.

In keeping with an aspect of the inventive process, the anionic dye and/or the di(4-chlorophenyldiguanido) compound of formula (I) or the pharmaceutically acceptable salt thereof can be dissolved in water before use. Preferably, the anionic dye is dissolved in water or in mixtures comprising water, and the di(4-chlorophenyldiguanido) compound of formula (I) or the pharmaceutically acceptable salt thereof is dissolved in isopropanol or in mixtures comprising isopropanol.

In an embodiment, the inventive process comprises mixing the one or more anionic dyes with water to form a solution and adding one or more C₁₋₈ alkanols to the solution.

The stable antiseptic solution of claim 10 comprising a di(4-chlorophenyldiguanido) compound of formula (I) or a pharmaceutically acceptable salt thereof, and one or more anionic dyes, wherein the anionic dye or the total amount of anionic dyes present in the antiseptic solution are in an amount sufficient to stain a patient's skin or surgical field before surgery, wherein the antiseptic solution is essentially free of any cationic excipient, can be prepared by a process wherein in step (i) one or more anionic dyes is dissolved in a portion of one more first solvents to provide an anionic dye solution, and finally the rest of the amount of the one or more first solvents is added to the solution (iii) formed after adding the di(4-chlorophenyldiguanido) compound of formula (I) or the pharmaceutically acceptable salt thereof.

In the process according to the present invention, the portion of solvent added to the anionic dye is typically at least 15% (v/v) of the required amount of solvent, preferably at least 30% (v/v), more preferred at least 50% (v/v), most preferred 80-90% (v/v), and still most preferred within the range 72 to 88% (v/v).

In keeping with the invention, the above solvents used can be the same or they can be different solvents. However, if the solvent for the anionic dye solution is only water, the di(4-chlorophenyldiguanido) compound of formula (I) or pharmaceutically acceptable salt thereof, is added dissolved in a solvent which is not only water. If the anionic dye is dissolved in a mixture of water and isopropanol, the di(4-chlorophenyldiguanido) compound of formula (I) or pharmaceutically acceptable salt thereof, is added dissolved in a solvent which is only isopropanol, or only water or a mixture of isopropanol with water.

It is preferred that a process according to the present invention comprises the steps of dissolving one or more anionic dyes in deionizied water to provide an anionic dye solution, then adding a portion of the amount of a C₂-C₈ lower alkanol, preferably isopropanol, then adding di(4-chlorophenyldiguanido) compound of formula (I) or a pharmaceutically acceptable salt thereof previously dissolved in water and mixing until complete dissolution, and finally adding the rest of the amount of the C₂-C₈ lower alkanol, preferably isopropanol.

Without wishing to be bound to a particular theory, it is believed that one or more complexes form between the components of the antiseptic solution (e.g., one or more anionic dyes, di(4-chlorophenyldiguanido) compound of formula (I) or pharmaceutically acceptable salt, and one or more solvents) which facilitate the chemical and physical stability of the compositions. For example, it is thought that the anionic dye forms a complex with solvent molecules when the anionic dye is dissolved in one or more first solvents, particularly when the first solvent comprises water or is water. Further, it is thought that the anionic dye-solvent complex is dispersed and is surrounded by a second solvent, when present, particularly when the second solvent comprises a C₂-C₈ lower alkanol or is a C₂-C₈ lower alkanol. It is also believed that the di(4-chlorophenyldiguanido) compound of formula (I) or pharmaceutically acceptable salt thereof forms a complex with the anionic dye complex, particularly if the di(4-chlorophenyldiguanido) compound of formula (I) or pharmaceutically acceptable salt thereof is added dispersed in a solvent (e.g., water), particularly in the presence of a C₂-C₈ lower alkanol. Complex formation occurs between the two systems due to the difference in solvent polarity that reaches stable state and ensures that the antiseptic solution of the present invention is chemically and physically stable and without precipitation or particle formation.

According to a further embodiment of the present invention, compound of formula (I) or a pharmaceutically acceptable salt thereof is separated in the stable antiseptic solution from the anionic dye by at least two solvents of different polarity. For example, the compound of formula (I) is essentially surrounded by one solvent and the anionic dye is essentially surrounded by the other solvent of different polarity.

It is further advantageous to prepare an antiseptic solution of the present invention under an inert atmosphere to help preserve the chemical and physical stability of the antiseptic solution. For example, the antiseptic solutions of the invention can be prepared under an inert gas atmosphere (e.g., nitrogen or argon gas). The skilled artisan will recognize that other variations are suitable.

Additionally, the process of the present invention may also comprise additional steps, for example, a final addition of more solvent.

In a preferred embodiment, the first solvent comprises one or more solvents. The first solvent comprises water or a mixture of water with one or more C₁₋₈ alkanols. The preferred C₁₋₈ alkanols are methanol, ethanol, propanol, isopropanol, butanol, pentanol, and mixtures thereof. In a particular preferred embodiment, the C₁₋₈ alkanol is isopropanol.

In keeping with an aspect of the invention, the step of providing an anionic dye solution can be divided into substeps. For example, in an embodiment the step of providing an anionic dye further comprises the steps of mixing the one or more anionic dyes with water to form a solution and adding one or more C₁₋₈ alkanols to the said solution. In a preferred embodiment, the lower alkanol is isopropanol.

When mixtures of water with one or more C₁₋₈ alkanols are used as a first solvent, the ratio of water to C₁₋₈ alkanol is at least 1:1 (v/v), that is, the amount of water in volume is equal to or greater than the amount of C₁₋₈ alkanol in volume. In a preferred embodiment, the ratio of water to C₁₋₈ alkanol is at least 2:1 (v/v), even more preferably at least 4:1 (v/v), still more preferably at least 8:1 (v/v), and most preferred the ratio of water and C₁₋₈ alkanol is at least 10:1 (v/v).

In a preferred embodiment the first solvent comprises more than 50% (v/v) of water. More preferably, the first solvent comprises more than 80% (v/v) of water. Most preferred, the first solvent is water.

In another preferred embodiment, the first solvent is a mixture of water and isopropanol.

The di(4-chlorophenyldiguanido) compound of formula (I) or pharmaceutically acceptable salt thereof, is added dissolved in one or more second solvents as defined in claim 1. Desirably, the anionic dye solution is stirred during the addition of the solution of di(4-chlorophenyldiguanido) compound of formula (I) or pharmaceutically acceptable salt thereof.

Just as the first solvent, the second solvent can comprise one or more solvents. The second solvent is selected from water, one or more C₁₋₈ alkanols, or a mixture thereof. The preferred C₁₋₈ alkanol of the second solvent is methanol, ethanol, propanol, isopropanol, butanol, pentanol, and mixtures thereof. Preferably, the second solvent comprises isopropanol.

In a preferred embodiment, the second solvent is water, methanol, ethanol, propanol, isopropanol, butanol, pentanol or any mixture thereof, preferably is water, isopropanol or water and isopropanol.

The second solvent can comprise a suitable amount of one or more C₁₋₈ alkanols. Preferably, the second solvent comprises more than 50% (v/v) of the total volume of the second solvent one or more C₁₋₈ alkanols, more preferably more than 80% (v/v) of one or more C₁₋₈ alkanols. In an even more preferred embodiment the second solvent is one or more C₁₋₈ alkanol. In a particularly preferred embodiment, the second solvent is isopropanol.

It is desirable that the first solvent comprises a suitable amount of water and that the second solvent comprises a suitable amount of one or more C₁₋₈ alkanols. In a preferred embodiment, the first solvent comprises more than 50% (v/v) of water, and the second solvent comprises more than 50% (v/v) of one or more C₁₋₈ alkanols. Even more preferably, the first solvent comprises more than 80% (v/v) of water and the second solvent comprises more than 80% (v/v) of one or more C₁₋₈ alkanols. Preferably, when the solvent for the anionic dye solution is only water, then the one or more second solvents comprise one or more C₁₋₈ alkanols (i.e., the second solvent is not only water). In addition, when the solvent for the anionic dye solution is isopropanol, then the second solvent is not only isopropanol.

In a preferred embodiment, the first solvent is water and the second solvent is a mixture of isopropanol and water.

In a preferred embodiment, the first solvent is water and the second solvent is isopropanol.

In a preferred embodiment, the first solvent is a mixture of water and isopropanol and the second solvent is isopropanol.

In a preferred embodiment, the first solvent is a mixture of water and isopropanol and the second solvent is another mixture of water and isopropanol.

In a preferred embodiment, the first solvent is a mixture of water and isopropanol and the second solvent is water.

In a particularly preferred embodiment, the inventive process provides an antiseptic solution comprising within the range 27 to 33% (v/v) of water, within the range 63 to 77% (v/v) of isopropanol, within the range 1.8 to 2.2% (w/v) of a pharmaceutically acceptable salt of the di(4-chlorophenyldiguanido) compound of formula (I), preferably the gluconate salt, and within the range 0.0225 to 0.0275% (w/v) of an anionic dye.

Another advantage is that the inventive process can be conducted at mild temperatures. For example, the anionic dye solution can be formed at a temperature below 60 ° C, preferably at a temperature below 40 ° C. In addition, the addition of the di(4-chlorophenyldiguanido) compound of formula (I) can be conducted at a temperature below 60 ° C, preferably at a temperature below 40 ° C.

In keeping with an aspect of the invention the additions of solvents during the inventive process can be made in a portion wise manner. For example, the anionic dye solution can be prepared such that only a portion of the first solvent is added during the early steps of the process. Accordingly, at a later time during the process the balance of the one or more first solvents can then be added. This is further illustrated as described herein.

In an embodiment, the invention provides a process for preparing a stable antiseptic solution of claim 10 comprising di(4-chlorophenyldiguanido) compound of formula (I) digluconate comprising the steps of (i) dissolving one or more anionic dyes in one or more first solvents, wherein the first solvent comprises water to provide an anionic dye solution, (ii) adding one or more C₁₋₈ alkanols to the anionic dye solution, (iii) dissolving the di(4-chlorophenyldiguanido) compound of formula (I) digluconate in one or more second solvents selected from the group consisting of C₁₋₈ alkanols, water and mixtures thereof to provide a solution of di(4-chlorophenyldiguanido) compound of formula (I) digluconate, (iv) combining the anionic dye solution and the solution of step (iii) to provide a combined solution, and (v) adding one or more lower alkanols to the combined solution of step (iv).

In a preferred embodiment, the antiseptic solution of claim 10 comprising di(4-chlorophenyldiguanido) compound of formula (I) or pharmaceutically acceptable salt thereof and an anionic dye in an amount sufficient to stain a patient's skin or surgical field before surgery, is obtainable by the process wherein after dissolving one or more anionic dyes in water until complete dissolution, a part of the amount of C₂-C₈ lower alkanol, preferably isopropanol is added followed by addition of solution of di(4-chlorophenyldiguanido) compound of formula (I) or pharmaceutically acceptable salt thereof and mixing until complete dissolution, and finally adding the rest of the amount of the C₂-C₈ lower alkanol, preferably isopropanol.

In a further particularly preferred embodiment, the antiseptic solution of claim 10 comprising di(4-chlorophenyldiguanido) compound of formula (I) digluconate thereof and FD&C Red No. 40 (Allura Red) anionic dye in an amount sufficient to stain a patient's skin or surgical field before surgery is obtainable by the process that comprises the steps of dissolving FD&C Red No. 40 (Allura Red) anionic dye in water until complete dissolution, and then adding 80% of the amount of isopropanol, then adding solution of di(4-chlorophenyldiguanido) compound of formula (I) digluconate and mixing until complete dissolution, and finally adding the rest of the amount of isopropanol.

An antiseptic solution as provided by the present invention is a disinfectant suitable for any purpose related to its antiseptic and/or disinfectant properties. For example, the inventive compositions can be used to prevent infections, including but not limited to, nosocomial infections, surgical-site infections, catheter-related infections, surgical wound infections, and oral infections. In addition, the antiseptic solutions of the invention can be used in, for example, skin disinfection; handwashing; oral care; irrigation of surgical wounds, the urinary bladder or vagina; the topical treatment of burn wounds; and the treatment of peritonitis in peritoneal dialysis.

### EXAMPLES

These following examples further illustrate the invention.

Amounts are reported in weight-volume percent, unless otherwise indicated. The stability tests were performed as described herein, unless otherwise noted.

### EXAMPLE 1

This example demonstrates stable antiseptic solutions of the invention, 1D and reference solutions 1A-1C and 1E, the components of which are listed in Table 1 and Table 2. Reference examples 1A-1C and example 1D contained FD&C Red No. 40 as the anionic dye and reference example 1E contained Food Red 3 Camoisina as the anionic dye. The antiseptic solutions contained isopropyl alcohol (1A), (1B) and 1D, 99% ethyl alcohol (1C), or 70% ethyl alcohol (1E) as the lower alkanols.

**TABLE 1**

| | **Ex. 1A*** | **Ex. 1B*** | **Ex. 1C*** | **Ex. 1D** |
|---|---|---|---|---|
| **Component** | **Amount (% w/v / v/v)** | | | |
| chlorhexidine digluconate (w/v) | 2 | 2 | 2 | 2 |
| anionic dye FD&C Red No. 40 (Allura Red) (w/v) | 0.2 | 0.45 | 0.2 | 0.025 |
| isopropyl alcohol (v/v) | 70 | 70 | -- | 70 |
| ethyl alcohol, 99% (v/v) | -- | -- | 70 | - |
| deionized water | qsp 100 mL | qsp 100 mL | qsp 100 mL | qsp 100 mL |

| | | | | |
|---|---|---|---|---|
| * - reference examples | | | | |

**TABLE 2**

| | **Ex. 1E*** |
|---|---|
| **Component** | **Amount** |
| chlorhexidine digluconate, 20% v/w, aq. solution | 1 mL |
| anionic dye Food Red 3 Camoisina | 0.07% (w/v) |
| ethyl alcohol, 70% | qsp 100 mL |

Antiseptic solutions 1A-1E were prepared as follows: anionic dye FD&C Red No. 40 (Allura Red) was dissolved in deionized water (1A-1D) and anionic dye Food Red 3 Carmoisine was dissolved in ethanol (Reference Example 1E). The mixtures were stirred with a magnetic stirrer until dissolution was complete at room temperature. 80% of theoretical weight of isopropanol (1A, 1B and 1D) and ethanol (1C and 1E) was added to the above solution and stirred with magnetic stirrer until complete homogenity for 10 minutes at room temperature. Chlorhexidine digluconate 20% v/w aqueous solution was weighed and added to the solutions IA to 1E. The mixture was stirred with magnetic stirrer until complete homogeneity for 10 minutes at room temperature. The remaining part of isopropanol (1A, 1B and 1D) and ethanol (1C and 1E) was added, and the mixture was stirred with magnetic stirrer for another 10 minutes at room temperature. The pH and density of the resulting solution were measured. The solution was filtered through clarificant filter of 100µm filters and filled in plastic high density polyethylene (HDPE) vials.

### EXAMPLE 2

This example demonstrates the stability of antiseptic solutions of the invention. The stability of the antiseptic solutions of Example 1 was evaluated at 0, 3, and 6 months using the following conditions: 25 ± 2 °C & 60% ± 5% relative humidity (Table 3), or 40 ± 2°C & 75% ± 5% relative humidity (Table 4). The visual appearance of the solutions was monitored for the presence of particles. The concentration of *p*-chloroaniline impurity was evaluated as described herein.

The results are set forth in Table 3 and Table 4.

**TABLE 3**

| **Comp.** | **Appearance (free of particles)** | | | **p-Chloroaniline [NMT 0.25%]** | | |
|---|---|---|---|---|---|---|
| | *0 months* | *3 months* | *6 months* | *0 months* | *3 months* | *6 months* |
| Ex. 1A* | conforms | conforms | conforms | 0.01% | 0.02% | 0.02% |
| Ex. 1B* | conforms | conforms | conforms | 0.01% | 0.01% | 0.02% |
| Ex. 1C* | conforms | conforms | conforms | 0.02% | 0.04% | 0.08% |
| Ex. 1D | conforms | - | - | 0.02% | - | - |
| Ex. 1E* | conforms | conforms | conforms | 0.02% | not tested | 0.06% |

| | | | | | | |
|---|---|---|---|---|---|---|
| * - comparative examples | | | | | | |

**TABLE 4**

| | | | | | | |
|---|---|---|---|---|---|---|
| **Comp.** | **Appearance (free of particles)** | | | **p-Chloroaniline [NMT 0.25%]** | | |

| | *0 months* | *3 months* | *6 months* | *0 months* | *3 months* | *6 months* |
|---|---|---|---|---|---|---|
| Ex. 1A* | conforms | conforms | conforms | 0.01% | 0.06% | 0.14% |
| Ex. 1B* | conforms | conforms | conforms | 0.01% | 0.08% | 0.12% |

As is apparent by the data set forth in Table 3 and Table 4, antiseptic solutions 1D in accordance with the invention have good chemical and physical stability properties.

## Claims

1. A process for preparing a colored antiseptic solution comprising a di(4-chlorophenyldiguanido) compound of formula (I) or a pharmaceutically acceptable salt thereof, 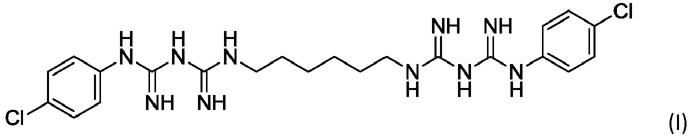 one or more anionic dyes, and one or more solvents, wherein the compound of formula (I) or a pharmaceutically acceptable salt thereof is present in an amount of 1% to 3% (w/v) and the one or more anionic dyes is present in an amount of 0.01% to 0.04% (w/v), the one or more solvents comprises water and from 20 to 95% (v/v) of one or more C₁₋₈ alkanols, wherein the antiseptic solution contains less than 0.01 wt % of cationic excipients and wherein the stable antiseptic solution is stable for at least 6 months at 25 ± 2 °C and 60% ± 5% relative humidity, or 40 ± 2 °C and 75% ± 5% relative humidity, comprising:
(i) dissolving one or more anionic dyes in one or more first solvents to provide an anionic dye solution, wherein the first solvent comprises water and optionally further comprises one or more C₁₋₈ alkanols,
(ii) dissolving the di(4-chlorophenyldiguanido) compound of formula (I) or a pharmaceutically acceptable salt thereof in one or more second solvents to provide a solution of a compound of formula (I) or a pharmaceutically acceptable salt thereof, wherein the second solvent is selected from the group consisting of one or more C₁₋₈ alkanols, water, and mixtures thereof, and
(iii) combining the anionic dye solution and solution of a compound of formula (I) or a pharmaceutically acceptable salt thereof,
with the proviso that if the solvent for the anionic dye solution is only water, then the one or more second solvents comprises one or more C₁₋₈ alkanols.

2. A process according to claim 1 further comprising, prior to step (ii), the step of adding one or more C₁₋₈ alkanols to the anionic dye solution.

3. A process according to claim 2 further comprising, after step (iii), the step of adding one or more C₁₋₈ alkanols to the combined solution of step (iii).

4. A process according to any of claims 1 to 3, wherein the first solvent comprises more than 50% (v/v) water.

5. A process according to any of claims 1 to 4, wherein the C₁₋₈ alkanol is ethanol, isopropanol or mixtures thereof.

6. A process according to any of claims 1 to 5, wherein the antiseptic solution comprises within the range 27 to 33% (v/v) of water, within the range 63 to 77% (v/v) of isopropanol, within the range 1.8 to 2.2% (w/v) of a pharmaceutically acceptable salt of a di(4-chlorophenyldiguanido) compound of formula (I), and within the range 0.0225 to 0.0275% (w/v) of an anionic dye.

7. A process according to any of claims 1 to 6, wherein the one or more anionic dye is selected from the group consisting of FD&C Blue No. 1 (erioglaucine disodium), FD&C Blue No. 2(3,3'-dioxo-2,2'-bis-indolyden-5,5'-disulfonic acid disodium salt), FD&C Green No. 3 (ethyl-[4-[[4-[ethyl-[(3-sulfophenyl) methyl]amino]phenyl]-(4-hydroxy-2-sulfophenyl)methylidene]-1-cyclohexa-2,5-dienylidene]-[(3-sulfophenyl)methyl]azanium, FD&C Red 40 (disodium 6-hydroxy-5-((2-methoxy-5-methyl-4-sulfophenyl)azo)-2-naphthalenesulfonate), Food Red 4 Carmoisine(disodium-4-hydroxy-2-[(E)-(4-sulfonato-1-naphthyl)diazenyl]naphthalene-1-sulfonate), FD&C Yellow No. 5((4E)-5-oxo-1-(4-sulfonatophenyl)-4-[(4-sulfonatophenyl)hydrazono]-3-pyrazolecarboxylate), FD&C Yellow No. 6 (disodium 6-hydroxy-5-[(4-sulfophenyl)azo]-2-naphthalenesulfononate), and mixtures thereof.

8. A process according to any of claims 1 to 7, wherein the pharmaceutically acceptable salt of said di(4-chlorophenyldiguanido) compound of formula (I) is N,N-bis(4-chlorophenyl)-3,12-diimino-2,4,11,13-tetraazatetradecanediimidamide digluconate.

9. A process according to any of claims 1 to 8, wherein the one or more anionic dye is FD&C Red 40 (disodium 6-hydroxy-5-((2-methoxy-5-methyl-4-sulfophenyl)azo)-2-naphthalenesulfonate)

10. A colored, stable antiseptic solution comprising a di(4-chlorophenyldiguanido) compound of formula (I) or a pharmaceutically acceptable salt thereof, 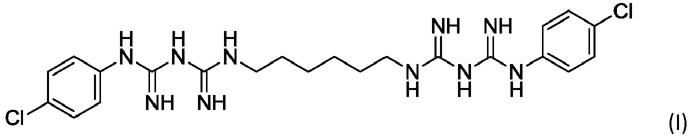 one or more anionic dyes, and one or more solvents, wherein the compound of formula (I) or a pharmaceutically acceptable salt thereof is present in an amount of 1% to 3% (w/v) and the one or more anionic dyes is present in an amount of 0.01% to 0.04% (w/v), the one or more solvents comprises water and from 20 to 95% (v/v) of one or more C₁₋₈ alkanols wherein the antiseptic solution contains less than 0.01 wt % of cationic excipients, wherein the stable antiseptic solution is stable for at least 6 months at 25 ± 2 °C and 60% ± 5% relative humidity, or 40 ± 2 °C and 75% ± 5% relative humidity, and wherein the stable antiseptic solution is prepared by the process according to claim 3.

11. A stable antiseptic solution according to claim 10, wherein the amount of p-chloroaniline impurity is less than 0.25% (w/w) for at least 6 months at 25 ± 2 °C and 60% ± 5% relative humidity, or 40 ± 2 °C and 75% ± 5 relative humidity.

12. A stable antiseptic solution according to claim 10 or 11, wherein the one or more C₁₋₈ alkanols is selected from the group consisting of isopropanol, ethanol and mixtures thereof.

13. A stable antiseptic solution according to any of claims 10 to 12, wherein the one or more C₁₋₈ alkanols is isopropanol.

14. A stable antiseptic solution according to any of claims 10 to 13, wherein the water is deionized water.

15. A stable antiseptic solution according to any of claims 10 to 14, wherein the solvent of the antiseptic solution is deionized water and 70% isopropanol.

## Patentansprüche

1. Verfahren zur Herstellung einer gefärbten antiseptische Lösung, die eine di(4-Chlorphenyldiguanid)-Verbindung der Formel (I) oder ein pharmazeutisch annehmbares Salz davon umfasst, 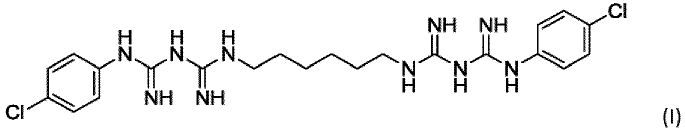 einen oder mehrere anionische Farbstoff(e) und eines oder mehrere Lösungsmittel, wobei die Verbindung der Formel (I) oder ein pharmazeutisch annehmbares Salz davon in einer Menge von 1 % bis 3 % (w/v) vorliegt und der eine oder die mehreren anionische(n) Farbstoff(e) in einer Menge von 0,01 % bis 0,04 % (w/v) vorliegt, das eine oder die mehreren Lösungsmittel Wasser umfasst bzw. umfassen und von 20 bis 95 % (v/v) von einem oder mehreren C₁₋₈-Alkanolen umfasst bzw. umfassen, wobei die antiseptische Lösung weniger als 0,01 Gew.-% an kationischen Hilfsstoffen enthält und wobei die stabile antiseptische Lösung für mindestens 6 Monate bei 25 ± 2 ° C und 60 % ± 5 % relativer Luftfeuchtigkeit oder 40 ± 2 ° C und 75 % ± 5 % relativer Luftfeuchtigkeit stabil ist, umfassend:
(i) Auflösen eines oder mehrerer anionischer Farbstoffe in einem oder mehreren ersten Lösungsmitteln zur Bereitstellung einer anionischen Farbstofflösung, wobei das erste Lösungsmittel Wasser und gegebenenfalls ferner ein oder mehrere C₁₋₈-Alkanole umfasst,
(ii) Auflösen der di(4-Chlorphenyldiguanid)-Verbindung der Formel (I) oder eines pharmazeutisch annehmbaren Salzes davon in einem oder mehreren zweiten Lösungsmitteln, um eine Lösung einer Verbindung der Formel (I) oder eines pharmazeutisch annehmbaren Salzes davon zur Verfügung zu stellen, wobei das zweite Lösungsmittel ausgewählt ist aus der Gruppe bestehend aus einem oder mehreren C₁₋₈-Alkanolen, Wasser und Mischungen davon, und
(iii) Kombinieren der anionischen Farbstofflösung und Lösung einer Verbindung der Formel (I) oder eines pharmazeutisch verträglichen Salzes davon,
mit der Maßgabe, dass, wenn das Lösungsmittel für die anionische Farbstofflösung nur Wasser ist, das eine oder die mehreren zweite(n) Lösungsmittel ein oder mehrere C₁₋₈-Alkanole enthält bzw. enthalten.

2. Verfahren nach Anspruch 1, ferner umfassend, vor Schritt (ii), den Schritt des Zugebens eines oder mehrere C₁₋₈-Alkanole zu der anionischen Farbstofflösung.

3. Verfahren nach Anspruch 2, ferner umfassend, nach Schritt (iii), den Schritt einer Zugabe von einem oder mehreren C₁₋₈-Alkanolen zu der kombinierten Lösung von Schritt (iii).

4. Verfahren nach einem der Ansprüche 1 bis 3, wobei das erste Lösungsmittel mehr als 50 % (v/v) Wasser enthält.

5. Verfahren nach einem der Ansprüche 1 bis 4, wobei das C₁₋₈-Alkanol Ethanol, Isopropanol oder Mischungen davon ist.

6. Verfahren nach einem der Ansprüche 1 bis 5, wobei die antiseptische Lösung Wasser im Bereich von 27 bis 33 % (v/v) aufweist, Isopropanol im Bereich von 63 bis 77 % (v/v), ein pharmazeutisch annehmbares Salz einer di(4-Chlorphenyldiguanid)-Verbindung der Formel (I) im Bereich von 1,8 bis 2,2 % (w/v) und einen anionischen Farbstoff im Bereich von 0,0225 bis 0,0275 % (w/v).

7. Verfahren nach einem der Ansprüche 1 bis 6, wobei der eine oder die mehreren anionische(n) Farbstoff(e) ausgewählt ist bzw. sind aus der Gruppe bestehend aus FD&C Blau Nr. 1 (Erioglaucindinatrium), FD&C Blau Nr. 2 (3,3'-Dioxo-2,2'-bis-indolyden-5,5'-disulfonsäure-dinatriumsalz), FD&C Grün Nr. 3 (Ethyl-[4-[[4-[ethyl-[(3-sulfophenyl)methyl]amino]phenyl]-(4-hydroxy-2-sulfophenyl)methyliden]-1-cyclohexa-2,5-dienyliden]-[(3-sulfophenyl)methyl]azan, FD&C Rot 40 (Dinatrium-6-hydroxy-5-((2-methoxy-5)-methyl-4-sulfophenyl)azo)-2-naphthalinsulfonat), Lebensmittelrot 4 Carmoisin (Dinatrium-4-hydroxy-2-[(E)-(4-sulfonat-1-naphthyl)diazenyl]naphthalin-1-sulfonat), FD&C Gelb Nr. 5 ((4E)-5-Oxo-1-(4-sulfonatophenyl)-4-[(4-sulfonatophenyl)hydrazon]-3-pyrazolcarboxylat), FD&C Gelb Nr. 6 (Dinatrium-6-hydroxy-5-[(4-sulfophenyl)azo]-2-naphthalinsulfononat) und Mischungen davon.

8. Verfahren nach einem der Ansprüche 1 bis 7, wobei das pharmazeutisch annehmbare Salz der di(4-Chlorphenyldiguanid)-Verbindung der Formel (I) N,N-bis(4-Chlorphenyl)-3,12-dümin-2,4,11,13-Tetraazatetradecandiimidamiddigluconat ist.

9. Verfahren nach einem der Ansprüche 1 bis 8, wobei der eine bzw. die mehreren anionische(n) Farbstoff(e) FD&C Rot 40 (Dinatrium-6-hydroxy-5-((2-methoxy-5-methyl-4-sulfophenyl) azo)-2-naphthalinsulfonat) ist bzw. sind.

10. Gefärbte, stabile antiseptische Lösung, die eine di(4-Chlorphenyldiguanid)-Verbindung der Formel (I) oder ein pharmazeutisch annehmbares Salz davon umfasst, 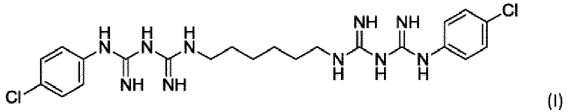 einen oder mehrere anionische Farbstoffe und eines oder mehrere Lösungsmittel, wobei die Verbindung der Formel (I) oder ein pharmazeutisch annehmbares Salz davon in einer Menge von 1 % bis 3 % (w/v) vorliegt und die einen oder mehrere anionische Farbstoffe in einer Menge von 0,01 % bis 0,04 % (w/v) vorliegen, das eine oder die mehreren Lösungsmittel Wasser und von 20 bis 95 % (v/v) von einem oder mehreren C₁₋₈-Alkanolen umfasst, wobei die antiseptische Lösung weniger als 0,01 Gew.-% an kationischem Exzipienten enthält, wobei die stabile antiseptische Lösung für mindestens 6 Monate bei 25 ± 2 ° C und bei 60 % ± 5 % relativer Luftfeuchtigkeit oder 40 ± 2 ° C und 75 % ± 5 % relativer Luftfeuchtigkeit stabil ist, und wobei die stabile antiseptische Lösung durch das Verfahren nach Anspruch 3 hergestellt wird.

11. Stabile antiseptische Lösung nach Anspruch 10, wobei die Menge an p-Chloranilin-Verunreinigung für mindestens 6 Monate bei 25 ± 2 ° C und 60 % ± 5 % relativer Luftfeuchtigkeit oder 40 ± 2 ° C und 75 % ± 5 relativer Luftfeuchtigkeit weniger als 0,25 % (w/w) beträgt.

12. Stabile antiseptische Lösung nach Anspruch nach Anspruch 10 oder 11, wobei das eine oder die mehreren C₁₋₈-Alkanole aus der Gruppe ausgewählt ist bzw. sind, bestehend aus Isopropanol, Ethanol und Mischungen davon.

13. Stabile antiseptische Lösung nach einem der Ansprüche 10 bis 12, wobei der eine oder die mehreren C₁₋₈-Alkanol(e) Isopropanol ist bzw. sind.

14. Stabile antiseptische Lösung nach einem der Ansprüche 10 bis 13, wobei das Wasser entionisiertes Wasser ist.

15. Stabile antiseptische Lösung nach einem der Ansprüche 10 bis 14, wobei das Lösungsmittel der antiseptischen Lösung entionisiertes Wasser und 70 % Isopropanol ist.

## Revendications

1. Procédé pour la préparation d'une solution antiseptique colorée comprenant un composé di (4-chlorophényldiguanido) de formule (I) ou un sel pharmaceutiquement acceptable de celui-ci, 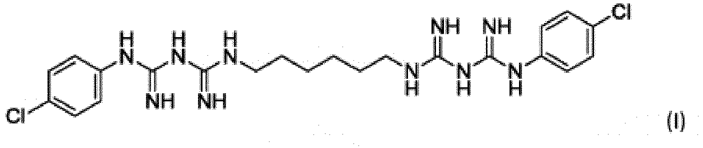 un ou plusieurs colorants anioniques, et un ou plusieurs solvants, dans lequel le composé de formule (I) ou un sel pharmaceutiquement acceptable de celui-ci est présent en une quantité comprise entre 1% et 3% (p/v) et le un ou plusieurs solvants est présent en une quantité comprise entre 0,01% et 0,04% (p/v), le un ou plusieurs solvants comprend l'eau et de 20 à 95% (v/v) de un ou plusieurs alcanols en C₁₋₈, dans lequel la solution antiseptique contient moins de 0,01% en poids d'excipients cationiques et dans lequel la solution antiseptique stable est stable pendant une durée d'au moins 6 mois à 25 ± 2°C et 60% ± 5% d'humidité relative ou 40 ± 2°C et 75% ± 5% d'humidité relative, comprenant:
(i) une dissolution d'un ou plusieurs colorants anioniques dans un ou plusieurs premiers solvants pour fournir une solution de colorant anionique, dans lequel le premier solvant comprend l'eau et comprend en outre optionnellement un ou plusieurs alcanols en C₁₋₈,
(ii) une dissolution du composé di (4-chlorophényldiguanido) de formule (I) ou un sel pharmaceutiquement acceptable de celui-ci dans un ou plusieurs seconds solvants pour fournir une solution d'un composé de formule (I) ou d'un sel pharmaceutiquement acceptable de celui-ci, dans lequel le second solvent est choisi dans le groupe constitué par un ou plusieurs alcanols en C₁₋₈, l'eau et leurs mélanges, et
(iii) une combinaison de la solution de colorant anionique et la solution d'un composé de formule (I) ou un sel pharmaceutiquement acceptable de celui-ci,
à la condition que si le solvent pour la solution de colorant anionique est l'eau seule, alors le un ou plusieurs seconds solvants comprend un ou plusieurs alcanols en C₁₋₈.

2. Procédé selon la revendication 1, comprenant en outre, avant l'étape (ii), l'étape d'une addition d'un ou plusieurs alcanols en C₁₋₈ la solution de colorant anionique.

3. Procédé selon la revendication 2, comprenant en outre, après l'étape (iii), l'étape d'une addition d'un ou plusieurs alcanols en C₁₋₈ à la solution combinée de l'étape (iii).

4. Procédé selon l'une quelconque des revendications 1 à 3, dans lequel le premier solvant comprend plus de 50% d'eau (v/v).

5. Procédé selon l'une quelconque des revendications 1 à 4, dans lequel l'alcanol en C₁₋₈ est l'éthanol, l'isopropanol ou leurs mélanges.

6. Procédé selon l'une quelconque des revendications 1 à 5, dans lequel la solution antiseptique comprend de l'eau dans la gamme de 27 à 33% (v/v), de l'isopropanol dans la gamme de 63 à 77% (v/v), un sel pharmaceutiquement acceptable du composé di (4-chlorophényldiguanido) de formule (I) dans la gamme de 1,8 à 2,2% (p/v), et un colorant anionique dans la gamme de 0,0225 à 0,0275% (p/v).

7. Procédé selon l'une quelconque des revendications 1 à 6, dans lequel le un ou plusieurs colorants anioniques est choisi dans le groupe constitué par le bleu No.1 FD&C (sel disodique d'érioglaucine), le bleu No.2 FD&C (sel disodique d'acide 3,3'-dioxo-2,2'-bis-indolydène-5,5'-disulfonique), vert No.3 FD&C (éthyl-[4-[[4-[éthyl-[(3-sulfophényl) méthyl] amino] phényl]-(4-hydroxy-2-sulfophényl) méthylidène]-1-cyclohexa-2,5-dienylidène]-[(3-sulfophényl) méthyl] azane, le rouge 40 FD&C (sel disodique du 6-hydroxy-5-((2-méthoxy-5-méthyl-4-sulfophényl) azo)-2-naphtalènesulfonate), le rouge alimentaire 4 Carmoisine (sel disodique du-4-hydroxy-2-[(E)-(4-sulfonato-1-naphtyl) diazenyl] naphtalène-1-sulfonate), jaune No.5 FD&C ((4E) -5-oxo-1-(4-sulfonatophényl)-4-[(4-sulfonatophényl) hydrazono]-3-pyrazolecarboxylate), le jaune No.6 FD&C (sel disodique du 6-hydroxy-5-[(4-sulfophényl) azo]-2-naphtalènesulfononate), et leurs mélanges.

8. Procédé selon l'une quelconque des revendications 1 à 7, dans lequel le sel pharmaceutiquement acceptable dudit composé di (4-chlorophényldiguanido) de formule (I) est le digluconate de N, N-bis (4-chlorophényl) -3,12-diimino-2, 4, 11, 13-tétraazatetradécanedümidamide.

9. Procédé selon l'une quelconque des revendications 1 à 8, dans lequel le un ou plusieurs colorants anioniques est le rouge 40 FD&C (sel disodique du 6-hydroxy-5-((2-méthoxy-5-méthyl- 4-sulfophényl) azo)-2-naphtalènesulfonate).

10. Solution antiseptique stable colorée comprenant un composé di (4-chlorophényldiguanido) de formule (I) ou un sel pharmaceutiquement acceptable de celui-ci, 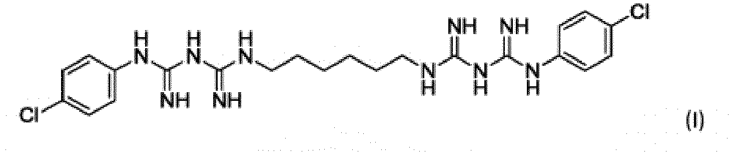 un ou plusieurs colorants anioniques, et un ou plusieurs solvants,
dans laquelle le composé di (4-chlorophényldiguanido) de formule (I) un sel pharmaceutiquement acceptable de celui-ci est présent en une quantité comprise entre 1% et 3%, (p/v) et le un ou plusieurs colorants anioniques est présent en une quantité comprise entre 0,01% et 0,04% (p/v), le un ou plusieurs solvants comprend l'eau et de 20 à 95% (v/v) de un ou plusieurs alcanols en C₁₋₈, dans laquelle la solution antiseptique contient moins de 0,01% en poids d'excipients cationiques et dans laquelle la solution antiseptique stable est stable pendant une durée d'au moins 6 mois à 25 ± 2°C et 60% ± 5% d'humidité relative, ou 40 ± 2°C et 75% ± 5% d'humidité relative, et dans laquelle la solution antiseptique stable est préparée par le procédé selon la revendication 3.

11. Solution antiseptique stable selon la revendication 10, dans laquelle la quantité d'impureté relative à la p-chloroaniline est de moins de 0,25% (p/p) pendant une durée d'au moins 6 mois à 25 ± 2°C et 60% ± 5% d'humidité relative ou 40 ± 2°C et 75% ± 5% d'humidité relative.

12. Solution antiseptique stable selon la revendication 10 ou 11, dans laquelle le un ou plusieurs alcanols en C₁₋₈ est choisi dans le groupe constitué par l'isopropanol, l'éthanol et leurs mélanges.

13. Solution antiseptique stable selon l'une quelconque des revendications 10 à 12, dans laquelle le un ou plusieurs alcanols en C₁₋₈ est l'isopropanol.

14. Solution antiseptique stable selon l'une quelconque des revendications 10 à 13, dans laquelle l'eau est l'eau désionisée.

15. Solution antiseptique stable selon l'une quelconque des revendications 10 à 14, dans laquelle le solvant de la solution antiseptique est l'eau désionisée et l'isopropanol à 70%.
